# EUROPEAN PATENT SPECIFICATION

(11) **EP 4 586 193 B1**
(45) Date of publication and mention of the grant of the patent: **16.09.2026**
(21) Application number: 25173322.6
(22) Date of filing: 12.06.2018
(51) Int. Cl.: A61B 8/14, A61B 8/08, G06T 7/00

(54) **ACOUSTIC WAVE DIAGNOSTIC APPARATUS AND CONTROL METHOD OF ACOUSTIC WAVE DIAGNOSTIC APPARATUS**
SCHALLWELLENDIAGNOSEVORRICHTUNG UND STEUERUNGSVERFAHREN EINER SCHALLWELLENDIAGNOSEVORRICHTUNG
APPAREIL DE DIAGNOSTIC À ONDES ACOUSTIQUES ET PROCÉDÉ DE COMMANDE D'APPAREIL DE DIAGNOSTIC À ONDES ACOUSTIQUES

(30) Priority: 25.08.2017 JP 2017162644
(43) Date of publication of application: 16.07.2025
(62) Document number(s) of the earlier application(s) in accordance with Art. 76 EPC: 18848765.6 / 3 673 816
(73) Proprietor: FUJIFILM Corporation, Tokyo 106-8620 (JP)
(72) Inventor: IMAI, Yoshiro, Ashigarakami-gun Kanagawa, 258-8538 (JP)
(74) Representative: Klunker IP Patentanwälte PartG mbB

(56) References cited:
- WO-A1-2016/190256
- JP-B1- 6 055 565
- US-A1- 2016 174 902

## Description

### BACKGROUND OF THE INVENTION

### 1. Field of the Invention

The present invention relates to an acoustic wave diagnostic apparatus and a control method of an acoustic wave diagnostic apparatus and in particular, to an acoustic wave diagnostic apparatus and a control method of an acoustic wave diagnostic apparatus for measuring a part on an acoustic wave image.

### 2. Description of the Related Art

In recent years, medical acoustic wave diagnostic apparatuses generally have a measurement function for measuring the length, size, area, and the like of various organs, lesions, and the like included in an acquired acoustic wave image. In order to measure a measurement target, a user usually operates a caliper, that is, a cursor using an input device for inputting coordinates, such as a track pad, a track ball, and a mouse, to set a measurement point, a region of interest, and the like on a display image. As described above, in a case where a manual operation is performed by the user, there is an influence due to the user's experience, skill level, and the like. Therefore, various attempts have been made to automate the operation.

For example, JP2010-240198A discloses an ultrasound diagnostic apparatus that, in a case where a body mark for a measurement target is selected by a user through an operation unit, automatically sets an image mode, image quality setting, a measurement mode, and a measurement item optimal for the measurement target.

In JP2010-240198A, for an ultrasound image, a measurement target is measured based on the position, number, and order of measurement points input from the user through the operation unit, and the measurement result is displayed on a display unit. JP2013-111434A discloses an ultrasound diagnostic apparatus that, in a case where a designation point is input on an ultrasound image from a user through an operation unit, determines an appropriate measurement point by performing image processing on a certain region centered on the input designation point. In JP2013-111434A, a measurement target is measured based on the measurement point determined in this manner.

JP6055565B1 discloses an ultrasonic diagnosis apparatus. US2016/174902A1 discloses a method and system for anatomical object detection using marginal space deep neural networks. WO2016/190256A1 discloses an ultrasound diagnostic device and image processing method.

### SUMMARY OF THE INVENTION

As described above, in the ultrasound diagnostic apparatuses disclosed in JP2010-240198A and JP2013-111434A, it is necessary to manually designate a measurement point and a designation point through the operation unit. Therefore, the measurement requires time and effort, and different measurement results may be obtained depending on the user.

The present invention has been made in order to solve such a conventional problem, and it is an object of the present invention to provide an acoustic wave diagnostic apparatus and a control method of an acoustic wave diagnostic apparatus capable of easily performing measurement and preventing differences in measurement results by users.

In order to achieve the aforementioned object, there is provided an acoustic wave diagnostic apparatus according to claim 1 and a control method of an acoustic wave diagnostic apparatus according to claim 18. Further preferred aspects are defined in dependent claims 2-17.

Since the acoustic wave diagnostic apparatus according to an aspect of the present invention comprises the measurement target determination unit that determines the measurement target included in the acoustic wave image displayed on the display unit and the measurement unit that measures the measurement target in the acoustic wave image based on the measurement algorithm and displays the measurement result on the display unit, it is possible to easily perform measurement and prevent differences in measurement results by users.

### BRIEF DESCRIPTION OF THE DRAWINGS

Fig. 1 is a block diagram showing the configuration of an ultrasound diagnostic apparatus according to a first embodiment of the present invention.
Fig. 2 is a block diagram showing the internal configuration of an image generation unit in the first embodiment of the present invention.
Fig. 3 is a flowchart showing a measurement operation of the ultrasound diagnostic apparatus according to the first embodiment of the present invention.
Fig. 4 is a diagram showing a display example of a measurement execution button in the first embodiment of the present invention.
Fig. 5 is a block diagram showing the configuration of an ultrasound diagnostic apparatus according to a second embodiment of the present invention.
Fig. 6 is a flowchart showing a measurement operation of the ultrasound diagnostic apparatus according to the second embodiment of the present invention.

### DESCRIPTION OF THE PREFERRED EMBODIMENTS

Hereinafter, embodiments of the present invention will be described with reference to the accompanying diagrams.

### First embodiment

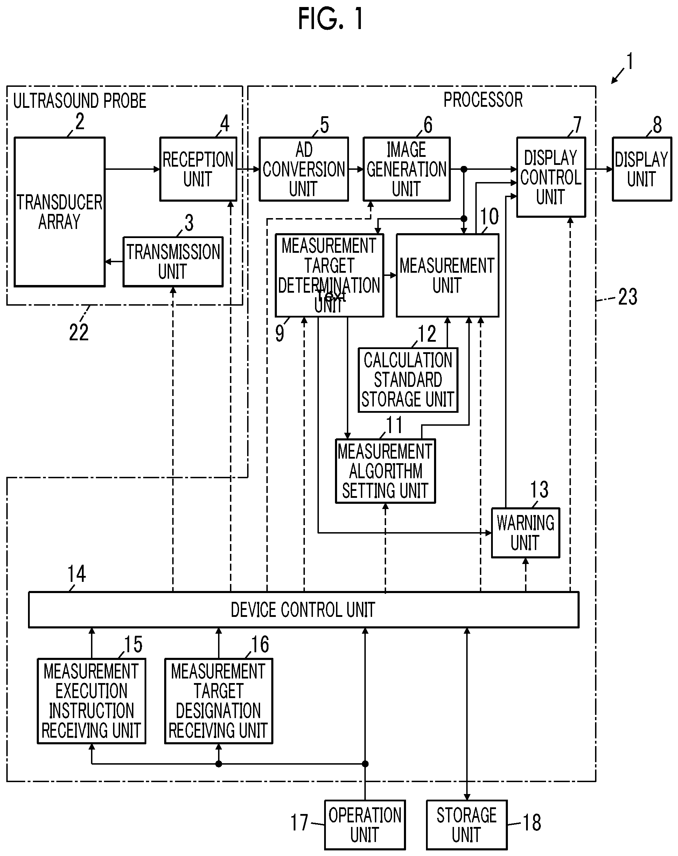

Fig. 1 shows the configuration of an ultrasound diagnostic apparatus 1 according to a first embodiment of the present invention. As shown in Fig. 1, the ultrasound diagnostic apparatus 1 comprises a transducer array 2, and a transmission unit 3 and a reception unit 4 are connected to the transducer array 2. An analog digital (AD) conversion unit 5, an image generation unit 6, a display control unit 7, and a display unit 8 are sequentially connected to the reception unit 4. A measurement target determination unit 9 and a measurement unit 10 are connected to the image generation unit 6, the measurement unit 10 is connected to the measurement target determination unit 9, and the display control unit 7 is connected to the measurement unit 10. A measurement algorithm setting unit 11 and a warning unit 13 are connected to the measurement target determination unit 9, the measurement unit 10 is connected to the measurement algorithm setting unit 11, and the display control unit 7 is connected to the warning unit 13. In addition, a calculation standard storage unit 12 is connected to the measurement unit 10.

A device control unit 14 is connected to the transmission unit 3, the reception unit 4, the image generation unit 6, the display control unit 7, the measurement target determination unit 9, the measurement unit 10, the measurement algorithm setting unit 11, and the warning unit 13. A measurement execution instruction receiving unit 15, a measurement target designation receiving unit 16, an operation unit 17, and a storage unit 18 are connected to the device control unit 14. The operation unit 17 is connected to the measurement execution instruction receiving unit 15 and the measurement target designation receiving unit 16.

An ultrasound probe 22 is configured by the transducer array 2, the transmission unit 3, and the reception unit 4, and a processor 23 is configured by the AD conversion unit 5, the image generation unit 6, the display control unit 7, the measurement target determination unit 9, the measurement unit 10, the measurement algorithm setting unit 11, the calculation standard storage unit 12, the warning unit 13, the device control unit 14, the measurement execution instruction receiving unit 15, and the measurement target designation receiving unit 16.

The transducer array 2 of the ultrasound probe 22 shown in Fig. 1 has a plurality of elements (ultrasound transducer) arranged in a one-dimensional or two-dimensional manner. According to a driving signal supplied from the transmission unit 3, each of the elements transmits an ultrasound wave and receives a reflected wave from a subject and outputs a reception signal. For example, each element is formed by using a transducer in which electrodes are formed at both ends of a piezoelectric body formed of piezoelectric ceramic represented by lead zirconate titanate (PZT), a polymer piezoelectric element represented by poly vinylidene di fluoride (PVDF), piezoelectric single crystal represented by lead magnesium niobate-lead titanate (PMN-PT), or the like.

The transmission unit 3 of the ultrasound probe 22 includes, for example, a plurality of pulse generators. Based on a transmission delay pattern selected according to the control signal from the device control unit 14, the transmission unit 3 adjusts the amount of delay of each driving signal so that ultrasound waves transmitted from the plurality of elements of the transducer array 2 form an ultrasound beam, and supplies the obtained signals to the plurality of elements. Thus, in a case where a pulsed or continuous-wave voltage is applied to the electrodes of the elements of the transducer array 2, the piezoelectric body expands and contracts to generate pulsed or continuous-wave ultrasound waves from each transducer. From the combined wave of these ultrasound waves, an ultrasound beam is formed.

The transmitted ultrasound beam is reflected by a target, for example, a part of the subject, and propagates toward the transducer array 2 of the ultrasound probe 22. The ultrasound waves propagating toward the transducer array 2 in this manner are received by the respective elements configuring the transducer array 2. In this case, the respective transducers configuring the transducer array 2 expand and contract by receiving the propagating ultrasound waves, thereby generating electric signals. These electric signals are output, as reception signals of the ultrasound waves, from each transducer to the reception unit 4. Although not shown, the reception unit 4 has an amplification unit for amplifying an ultrasound reception signal input from each transducer. In a case where the amplified signal is converted into digitized element data by the AD conversion unit 5, the element data is output to the image generation unit 6.

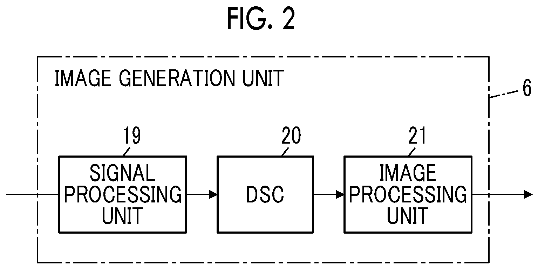

As shown in Fig. 2, the image generation unit 6 of the processor 23 has a configuration in which a signal processing unit 19, a digital scan converter (DSC) 20, and an image processing unit 21 are connected in series to each other. Based on a reception delay pattern selected according to the control signal from the device control unit 14, the signal processing unit 19 performs reception focusing processing in which delays are given to respective pieces of element data according to the set sound speed and addition (phasing addition) is performed. Through the reception focusing processing, a sound ray signal with narrowed focus of the ultrasound echo is generated. The signal processing unit 19 generates a B mode image signal, which is tomographic image information regarding tissues inside the subject, by correcting the attenuation of the generated sound ray signal due to the propagation distance according to the depth of the reflection position of the ultrasound wave and then performing envelope detection processing. The B mode image signal generated as described above is output to the DSC 20.

The DSC 20 raster-converts the B mode image signal into an image signal according to the normal television signal scanning method. The image processing unit 21 performs various kinds of necessary image processing, such as brightness correction, gradation correction, sharpness correction, and color correction, on the image data obtained in the DSC 20, and then outputs the B mode image signal to the display control unit 7, the measurement target determination unit 9, and the measurement unit 10. Hereinafter, the B mode image signal is referred to as an ultrasound image.

The measurement target determination unit 9 of the processor 23 determines a measurement target included in the ultrasound image by performing image recognition on the ultrasound image generated by the image generation unit 6. Here, the measurement target can include a measurement target part, such as an organ, and a lesion part, such as a tumor, a cyst, and bleeding. The measurement target determination unit 9 can determine a measurement target in the ultrasound image using machine learning, such as deep learning, for example. In this case, for example, by causing the measurement target determination unit 9 to learn a large amount of typical pattern data for the measurement target as positive data in advance and learn a large amount of pattern data other than the typical pattern data for the measurement target as negative data in advance, a neural network can be constructed. The measurement target determination unit 9 can determine a measurement target by calculating the length or the like of a characteristic portion for patterns included in the ultrasound image and classifying the patterns into learned pattern data using the calculation result and the constructed neural network.

In this case, the measurement target determination unit 9 can determine the measurement target by assigning the likelihood for the learned pattern data to the pattern included in the ultrasound image and performing threshold value determination for the likelihood. Here, the likelihood is a value indicating the likelihood of the pattern included in the ultrasound image for a plurality of pieces of learned pattern data. For example, in a case where the likelihood of the pattern included in the ultrasound image with respect to the pattern data of the gallbladder is high, the probability that the pattern included in the ultrasound image will be the gallbladder is high.

For example, the measurement target determination unit 9 can determine a measurement target by storing typical pattern data in advance as a template, calculating a pattern data similarity while searching for an image with a template, and considering that a measurement target is present in a place where the similarity is equal to or greater than a threshold value and is the maximum. For the calculation of the similarity, in addition to simple template matching, for example, a machine learning method described in Csurka et al.: Visual Categorization with Bags of Keypoints, Proc. of ECCV Workshop on Statistical Learning in Computer Vision, pp. 59-74 (2004) can be used.

In this manner, the measurement target determination unit 9 can determine the measurement target in the ultrasound image. The determination of the measurement target includes a case where the pattern in the ultrasound image is determined as only one measurement target, a case where the pattern in the ultrasound image is not determined as a measurement target, and a case where the pattern in the ultrasound image is determined as a plurality of measurement targets, such as gallbladder and abdominal aorta, according to the score assigned to the pattern in the ultrasound image by the measurement target determination unit 9.

As a case where it is determined that the pattern in the ultrasound image is not determined as a measurement target, for example, a case can be mentioned in which all of the scores of likelihood, similarity, and the like assigned to the pattern in the ultrasound image for a plurality of measurement target candidates are equal to or less than a predetermined threshold value. As a case where it is determined that the pattern in the ultrasound image is determined as a plurality of measurement targets, for example, a case can be mentioned in which some of the scores of likelihood, similarity, and the like assigned to the pattern in the ultrasound image for a plurality of measurement target candidates exceed a predetermined threshold value.

The measurement algorithm setting unit 11 of the processor 23 sets a measurement algorithm for the measurement target determined by the measurement target determination unit 9 and the measurement target whose designation from the user is received by the measurement target designation receiving unit 16 through the operation unit 17 as will be described later. The measurement algorithm setting unit 11 stores an algorithm corresponding to each measurement target as an association table, and sets a measurement algorithm with reference to the association table in a case where the measurement target is determined.

Here, there are generally different measurement rules for each measurement target. The measurement rule is a rule regarding which part is to be measured and how the part is to be measured with respect to a specific measurement target. For example, in a case where the measurement target is the gallbladder, the measurement rule is that a line segment, which has two points on the inner wall of the gallbladder region included in the ultrasound image as its end points, passes through the center of gravity of the gallbladder region, and has a maximum distance, is determined as a measurement line and the length of the determined line segment is measured. In addition, for example, in a case where the measurement target is the kidney, the measurement rule defines that the length between two points having a maximum distance therebetween among two points on the boundary of the kidney region included in the ultrasound image is measured. The measurement algorithm defines calculation means for executing such measurement rules, and differs for each measurement target.

Here, the algorithm defines calculation means for achieving the purpose, such as measurement. For example, the algorithm is implemented as a software program in an apparatus and is executed by a central processing unit (CPU). As the measurement algorithm set in the measurement algorithm setting unit 11, a known algorithm that is generally used can be used.

The measurement unit 10 of the processor 23 performs measurement based on the measurement algorithm set by the measurement algorithm setting unit 11 for the measurement target determined by the measurement target determination unit 9 and the measurement target whose designation from the user is received by the measurement target designation receiving unit 16 through the operation unit 17 as will be described later, and the measurement result is displayed on the display unit 8 through the display control unit 7. Here, the measurement result displayed on the display unit 8 by the measurement unit 10 may include the name of the measurement target and the measurement line used for measurement in addition to the measurement value for the measurement target. In the case of performing measurement on the measurement target, the measurement unit 10 calculates a measurement value based on the calculation standard stored in advance in the calculation standard storage unit 12.

In a case where it is determined that no measurement target is determined by the measurement target determination unit 9 and a case where a plurality of measurement targets are determined by the measurement target determination unit 9, the warning unit 13 of the processor 23 issues a warning and causes the display control unit 7 to display a plurality of measurement target candidates or the plurality of determined measurement targets on the display unit 8. For example, in a case where it is determined that no measurement target is determined by the measurement target determination unit 9, the warning unit 13 can display a text indicating that no measurement target is found on the display unit 8 and display a plurality of predetermined measurement target candidates on the display unit 8. In addition, for example, in a case where it is determined that a plurality of measurement targets are determined by the measurement target determination unit 9, the warning unit 13 can display a text indicating that a plurality of measurement targets have been found on the display unit 8 and display a plurality of determined measurement targets on the display unit 8 as measurement target candidates.

The warning unit 13 can emit sound indicating the warning content instead of the text indicating the warning content, or can emit sound while displaying the text indicating the warning content on the display unit 8.

The measurement execution instruction receiving unit 15 of the processor 23 receives a measurement execution instruction for the ultrasound image displayed on the display unit 8 from the user through the operation unit 17.

In a case where a plurality of measurement target candidates and a plurality of measurement targets determined by the measurement target determination unit 9 are displayed on the display unit 8, the measurement target designation receiving unit 16 of the processor 23 receives designation of a measurement target that is selected from the plurality of measurement target candidates by the user through the operation unit 17.

The display control unit 7 of the processor 23 generates a composite image by combining the image data generated by the image generation unit 6 with the measurement value calculated by the measurement unit 10, and displays the composite image on the display unit 8.

The display unit 8 of the ultrasound diagnostic apparatus 1 includes, for example, a display device, such as a liquid crystal display (LCD), and displays an ultrasound image under the control of the device control unit 14. The operation unit 17 of the ultrasound diagnostic apparatus 1 is for the user to perform an input operation, and can be configured to comprise a keyboard, a mouse, a track pad, a touch panel, and the like.

The storage unit 18 of the ultrasound diagnostic apparatus 1 stores an operation program and the like of the ultrasound diagnostic apparatus 1, and recording media, such as a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), and a universal serial bus memory (USB memory), or a server can be used.

The AD conversion unit 5, the image generation unit 6, the display control unit 7, the measurement target determination unit 9, the measurement unit 10, the measurement algorithm setting unit 11, the calculation standard storage unit 12, the warning unit 13, the device control unit 14, the measurement execution instruction receiving unit 15, and the measurement target designation receiving unit 16 are configured by a CPU and a control program causing the CPU to execute various kinds of processing. However, these may also be configured by digital circuits. The AD conversion unit 5, the image generation unit 6, the display control unit 7, the measurement target determination unit 9, the measurement unit 10, the measurement algorithm setting unit 11, the calculation standard storage unit 12, the warning unit 13, the device control unit 14, the measurement execution instruction receiving unit 15, and the measurement target designation receiving unit 16 can also be integrated partially or entirely into one CPU.

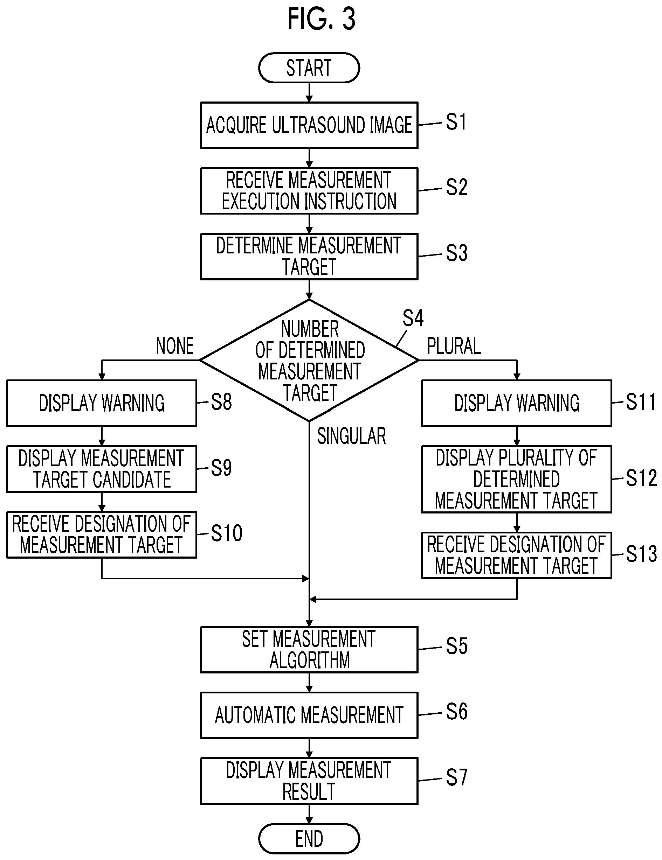

Next, the measurement operation of the ultrasound diagnostic apparatus 1 according to the first embodiment will be described with reference to the flowchart shown in Fig. 3.

First, in step S1, the ultrasound diagnostic apparatus 1 acquires an ultrasound image, and the acquired one ultrasound image is displayed on the display unit 8. As this ultrasound image, an image captured on the spot using the ultrasound probe 22 can be used. In this case, the user can display one ultrasound image on the display unit 8 by freezing ultrasound images, which are continuously captured by the ultrasound probe 22 and sequentially displayed on the display unit 8, through the operation unit 17. In addition, as an ultrasound image, an image acquired from an external memory (not shown) can also be used.

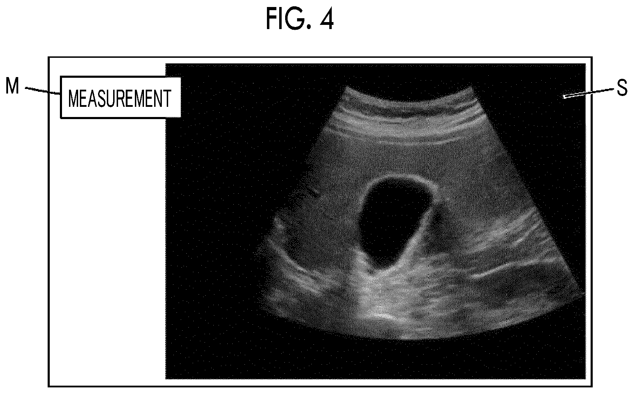

Then, in step S2, the measurement execution instruction receiving unit 15 receives a measurement execution instruction for the acquired ultrasound image from the user through the operation unit 17. For example, as shown in Fig. 4, in a case where a measurement execution button M is displayed on the display unit 8 together with an ultrasound image S and the measurement execution button M is selected from the user through the operation unit 17, the measurement execution instruction receiving unit 15 can receive a measurement execution instruction for the ultrasound image S.

In subsequent step S3, the measurement target determination unit 9 determines a measurement target included in the ultrasound image displayed on the display unit 8 by image recognition. In a case where the measurement target determination operation of the measurement target determination unit 9 ends, the process proceeds to step S4.

In step S4, the measurement target determination unit 9 determines whether or not only one measurement target has been determined in step S3, or determines whether or not no measurement target has been determined, or determines whether or not a plurality of measurement targets have been determined. Here, in a case where it is determined that only one measurement target has been determined in step S3, the process proceeds to step S5.

In step S5, the measurement algorithm setting unit 11 sets a measurement algorithm for only one measurement target determined in step S3. For example, in a case where the measurement target is the gallbladder, the measurement algorithm setting unit 11 determines a maximum distance line segment, which has two points disposed on the inner wall of the gallbladder region as end points, as a measurement line, and set a measurement algorithm for measuring the length of the measurement line. In addition, for example, in a case where the measurement target is the abdominal aorta, the measurement algorithm setting unit 11 sets a measurement algorithm for extracting the contour line of the outer wall of the abdominal aorta as a measurement line, calculating the total number of pixels inside the extracted contour line, and converting the calculated total number of pixels into the cross-sectional area of the abdominal aorta. The measurement algorithm setting unit 11 can also set the measurement algorithm so as to measure both the length and the area according to the measurement target.

In subsequent step S6, the measurement unit 10 performs automatic measurement on the measurement target based on the set measurement algorithm. In a case where the measurement value for the measurement target is calculated by the automatic measurement, the measurement unit 10 displays the measurement result on the display unit 8. In a case where step S7 ends, the measurement operation of the ultrasound diagnostic apparatus 1 ends.

In a case where it is determined that no measurement target has been determined by the measurement target determination unit 9 in step S4, the process proceeds to step S8.

In step S8, the warning unit 13 displays a warning display indicating that no measurement target has been determined on the display unit 8.

In subsequent step S9, the warning unit 13 displays a plurality of predetermined measurement target candidates on the display unit 8. In this case, in the measurement target determination operation in step S3, the warning unit 13 can display a plurality of measurement target candidates on the display unit 8 as a list in descending order of the scores of likelihood, similarity, and the like assigned to the pattern included in the ultrasound image for the plurality of measurement target candidates.

In step S10, the measurement target designation receiving unit 16 receives designation of the measurement target selected by the user through the operation unit 17 from the plurality of measurement target candidates displayed on the display unit 8 in step S9. In a case where the designation of the measurement target is received in this manner, the process proceeds to step S5 in which a measurement algorithm is set for the designated measurement target. In a case where the automatic measurement for the measurement target is performed in step S6, the measurement result is displayed on the display unit 8 in step S7. In this manner, the measurement operation in the ultrasound diagnostic apparatus 1 ends.

In a case where it is determined that a plurality of measurement targets have been determined by the measurement target determination unit 9 in step S4, the process proceeds to step S11.

In step S11, the warning unit 13 displays a warning display indicating that a plurality of measurement targets have been determined on the display unit 8.

In subsequent step S12, the warning unit 13 displays the plurality of measurement targets determined by the measurement target determination unit 9 on the display unit 8. In this case, in the measurement target determination operation in step S3, the warning unit 13 can display a plurality of measurement targets on the display unit 8 as a list in descending order of the scores of likelihood, similarity, and the like assigned to the pattern included in the ultrasound image for the plurality of measurement targets.

In step S13, the measurement target designation receiving unit 16 receives designation of the measurement target selected by the user through the operation unit 17 from the plurality of measurement targets displayed on the display unit 8 in step S12. In a case where the designation of the measurement target is received in this manner, the process proceeds to step S5 in which a measurement algorithm is set for the designated measurement target. In a case where the automatic measurement for the measurement target is performed in step S6, the measurement result is displayed on the display unit 8 in step S7. In this manner, the measurement operation in the ultrasound diagnostic apparatus 1 ends.

According to the ultrasound diagnostic apparatus 1 of the first embodiment described above, the measurement execution instruction receiving unit 15 receives a measurement execution instruction given from the user through the operation unit 17, the measurement target in the ultrasound image is automatically determined by the measurement target determination unit 9, and measurement corresponding to the determined measurement target is automatically made. Therefore, measurement can be easily performed without requiring the user's time and effort. In addition, as described above, the determination and measurement of the measurement target are automatically performed, and the user's operation through the operation unit 17 does not contribute to the measurement result. Therefore, differences in measurement results by users can be prevented.

The ultrasound diagnostic apparatus 1 according to the first embodiment of the present invention performs measurement of a measurement target on the ultrasound image. However, the measurement may also be performed on an acoustic wave image other than the ultrasound image. For example, also for a photoacoustic wave image and a composite image obtained by superimposing an ultrasound image and a photoacoustic wave image, measurement of the measurement target may also be performed.

In the first embodiment, the measurement target determination unit 9 determines the measurement target included in the ultrasound image with reference to the ultrasound image displayed on the display unit 8. However, the measurement target included in the ultrasound image may be determined with reference to a plurality of ultrasound images. For example, the measurement target determination unit 9 may determine the measurement target by performing image recognition on the ultrasound image displayed on the display unit 8 and ultrasound images of the preceding and subsequent frames. Here, the ultrasound images of the preceding and subsequent frames are ultrasound images captured before and after the ultrasound image displayed on the display unit 8 in time series. The number of ultrasound images of the preceding and subsequent frames referred to by the measurement target determination unit 9 is not particularly limited, and at least one of the ultrasound images of the preceding frames or the ultrasound image of the subsequent frames with respect to the ultrasound image displayed on the display unit 8 may be used or a plurality of ultrasound images of the preceding frames and a plurality of ultrasound images of the subsequent frames with respect to the ultrasound image displayed on the display unit 8 may be used.

In addition, although the measurement algorithm setting unit 11 automatically sets a measurement algorithm according to the measurement target, the measurement algorithm to be set may be set in advance according to the user's preference or the like. For example, a plurality of measurement algorithms may be stored in advance in the storage unit 18 or the like for one measurement target, and a measurement algorithm corresponding to the measurement target may be selected in advance by the user through the operation unit 17. For example, a measurement algorithm for measuring the length of the short axis of the gallbladder and a measurement algorithm for measuring the length of the long axis of the gallbladder in a case where the measurement target is the gallbladder may be stored in advance in the storage unit 18 or the like, and the measurement algorithm for measuring the length of the short axis may be selected in advance by the user in a case where the gallbladder is a measurement target. In this case, the measurement algorithm setting unit 11 automatically selects the measurement algorithm for measuring the length of the short axis of the gallbladder in a case where the measurement target is the gallbladder.

In the measurement operation in the first embodiment, the measurement target determination of the measurement target determination unit 9 is started with the reception of the measurement execution instruction by the measurement execution instruction receiving unit 15, which is given by the user through the operation unit 17, in step S2 as a trigger. However, the present invention is not limited to this. For example, in a case where ultrasound images are continuously captured by the ultrasound probe 22, the measurement target determination of the measurement target determination unit 9 may be started with the freezing of the ultrasound images sequentially displayed on the display unit 8, which is performed by the user through the operation unit 17, and the display of one ultrasound image on the display unit 8 as a trigger. Alternatively, the measurement target determination of the measurement target determination unit 9 may be started with the display of an ultrasound image stored in a memory, such as an external memory (not shown), on the display unit 8 as a trigger, for example.

In this manner, since the user's operation through the operation unit 17 can be further reduced, it is possible to perform measurement more easily.

In the case of displaying the measurement result on the display unit 8 in step S7, in a case where the measurement result includes at least one of the measurement line, the name of the measurement target, or the like in addition to the measurement value, the measurement unit 10 may display the measurement result on the display unit 8 by changing at least one of the color, thickness, line type such as solid line and broken line, or transparency of the measurement value and relevant items, such as a measurement value and a corresponding measurement line, a measurement value and the name of a corresponding measurement target, and a measurement value, a corresponding measurement line, and the name of a measurement target.

In the case of displaying a plurality of measurement target candidates on the display unit 8 in step S9, the warning unit 13 may display the plurality of measurement target candidates in a predetermined order. Similarly, in the case of displaying a plurality of measurement targets on the display unit 8 in step S12, the warning unit 13 may display the plurality of measurement targets in a predetermined order. In addition, in the case of displaying a plurality of measurement targets on the display unit 8 in step S12, the warning unit 13 may display the plurality of measurement targets in different colors.

In a case where the number of the plurality of measurement target candidates displayed on the display unit 8 in step S9 and the number of the plurality of measurement targets displayed on the display unit 8 in step S12 are large, an upper limit may be set for the number of measurement target candidates and the number of measurement targets displayed at one time on the display unit 8 in order to prevent the display on the display unit 8 from becoming complicated. For example, only a predetermined number of measurement target candidates and only a predetermined number of measurement targets may be displayed on the display unit 8, and the measurement target candidates and the measurement targets may be appropriately switched and displayed by the user operation through the operation unit 17.

### Second embodiment

In the measurement operation in the first embodiment, the measurement operation ends by displaying the measurement result on the display unit 8. In the measurement operation in the second embodiment, manual correction by the user through the operation unit 17 may be received for the measurement result displayed on the display unit 8.

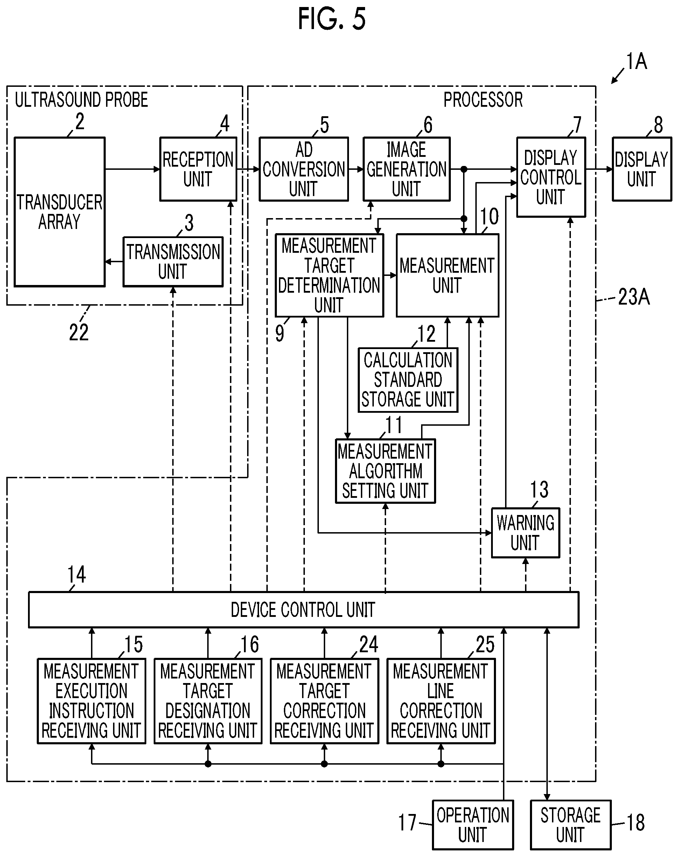

Fig. 5 shows the configuration of an ultrasound diagnostic apparatus 1A according to a second embodiment. The ultrasound diagnostic apparatus 1A according to the second embodiment is the same as the ultrasound diagnostic apparatus 1 according to the first embodiment except that a processor 23A has a measurement target correction receiving unit 24 and a measurement line correction receiving unit 25.

The measurement target correction receiving unit 24 of the processor 23A receives the correction of the measurement target from the user through the operation unit 17 for the measurement result displayed on the display unit 8. For example, in a case where the measurement result displayed on the display unit 8 is a measurement result for the gallbladder and the measurement target is corrected to a measurement target other than the gallbladder such as the abdominal aorta, the measurement target correction receiving unit 24 receives the correction of the measurement target from the user through the operation unit 17.

The measurement line correction receiving unit 25 of the processor 23A receives the correction of the measurement line and the correction result from the user through the operation unit 17 for the measurement result displayed on the display unit 8. For example, in a case where the display result displayed on the display unit 8 is the gallbladder and the measurement line is corrected without correcting the measurement target itself, the measurement line correction receiving unit 25 receives the correction of the measurement line from the user through the operation unit 17.

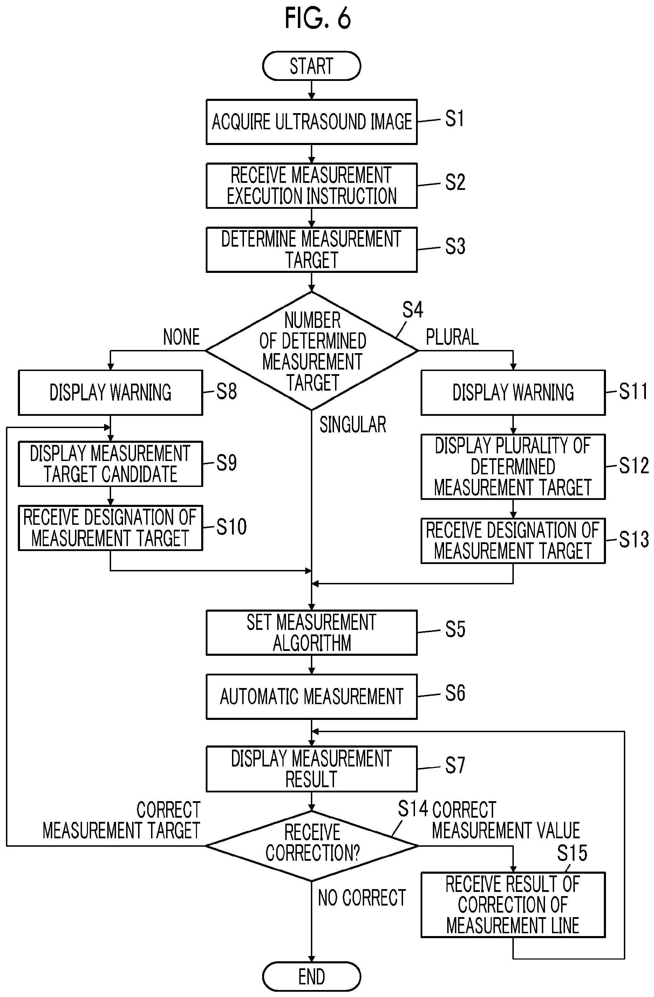

Next, the measurement operation of the ultrasound diagnostic apparatus 1A in the second embodiment will be described with reference to the flowchart shown in Fig. 6. Steps S1 to S7, steps S8 to S10, and steps S11 to S13 are the same as steps S1 to S7, steps S8 to S10, and steps S11 to S13 in the first embodiment shown in Fig. 3.

In a case where the measurement result is displayed on the display unit 8 in step S7, the process proceeds to step S14.

In step S14, the measurement target correction receiving unit 24 receives the correction of the measurement target from the user through the operation unit 17 for the measurement result displayed on the display unit 8. In step S14, the measurement line correction receiving unit 25 receives the correction of the measurement line from the user through the operation unit 17 for the measurement result displayed on the display unit 8.

In a case where the correction of the measurement target is received in step S14, the process returns to step S9 to display a plurality of measurement target candidates. In subsequent step S10, the measurement target designated by the user through the operation unit 17 is received. For example, by displaying the names of a plurality of measurement target candidates on the display unit 8 so that the user selects one of the measurement target candidates, the measurement target correction receiving unit 24 receives the correction of the measurement target.

In a case where the measurement target is received in step S10 a measurement algorithm is set, automatic measurement is performed on the measurement target, and the measurement result for the corrected measurement target is displayed on the display unit 8 in steps S5 to S7, and the process proceeds to step S14.

In a case where the correction of the measurement line is received in step S14, the process proceeds to step S15. For example, by displaying the measurement line on the display unit 8 so that the measurement line is manually adjusted by the user through the operation unit 17, the measurement line correction receiving unit 25 can receive the correction of the measurement line. For example, in a case where the measurement line is a line segment, the measurement line is corrected by adjusting the position of the end point of the line segment. In this case, a measurement value for the corrected measurement line is calculated.

In step S15, the measurement line correction receiving unit 25 receives the result of the manual correction of the measurement line made by the user through the operation unit 17, and the process returns to step S7 in which the manually corrected measurement line and the corresponding measurement value are displayed on the display unit 8. Then, the process proceeds to step S14.

In a case where there is no correction of the measurement target and the measurement line in step S14, the measurement operation in the ultrasound diagnostic apparatus 1A ends.

As described above, by receiving the correction of the measurement target and the measurement line in step S14 subsequent to step S7, the reliability of the measurement result finally obtained in the ultrasound diagnostic apparatus 1A can be improved.

In a case where the measurement target correction is received in step S14 and a plurality of measurement target candidates are displayed on the display unit 8 in step S9, the warning unit 13 may display measurement target candidates other than the measurement target, which has been automatically measured in step S6, on the display unit 8.

Similarly to step S9 in the first embodiment, the warning unit 13 may display a plurality of measurement target candidates as a list on the display unit 8 in descending order of the scores of likelihood, similarity, and the like assigned to the pattern included in the ultrasound image in step S3, or may display a plurality of measurement target candidates as a list on the display unit 8 in a predetermined order.

### Explanation of References

1, 1A: ultrasound diagnostic apparatus
2: transducer array
3: transmission unit
4: reception unit
5: AD conversion unit
6: image generation unit
7: display control unit
8: display unit
9: measurement target determination unit
10: measurement unit
11: measurement algorithm setting unit
12: calculation standard storage unit
13: warning unit
14: device control unit
15: measurement execution instruction receiving unit
16: measurement target designation receiving unit
17: operation unit
18: storage unit
19: signal processing unit
20: DSC
21: image processing unit
22: ultrasound probe
23, 23A: processor
24: measurement target correction receiving unit
25: measurement line correction receiving unit
M: measurement execution button
S: ultrasound image

## Claims

1. An acoustic wave diagnostic apparatus, comprising:
an operation unit (17) for a user to perform an input operation;
a measurement execution instruction receiving unit (15) that receives a measurement execution instruction from the user through the operation unit;
a display unit (8) that displays an acquired acoustic wave image;
a measurement target determination unit (9) that determines a measurement target included in the acoustic wave image displayed on the display unit by using machine learning, the measurement target including an organ or a lesion part;
a measurement algorithm setting unit (11) that sets a measurement algorithm based on the measurement target; and
a measurement unit (10) that measures the measurement target in the acoustic wave image based on the measurement algorithm set by the measurement algorithm setting unit and displays a measurement result on the display unit;
wherein the measurement target determination unit (9) determines the measurement target using machine learning by assigning a likelihood for learned pattern data to the pattern included in the acoustic wave image and performing threshold value determination for the likelihood, the likelihood being a value indicating the likelihood of the pattern included in the acoustic wave image for a plurality of pieces of learned pattern data.

2. The acoustic wave diagnostic apparatus according to claim 1,
wherein, in case where it is determined that the pattern in the acoustic wave image is determined as a plurality of measurement targets, it is determined that a pattern in the acoustic wave image is determined as a plurality of measurement targets in a case where the likelihoods assigned to the pattern for the plurality of measurement targets exceed a predetermined threshold value.

3. The acoustic wave diagnostic apparatus according to any of claims 1 to 2,
wherein the measurement target determination unit determines the measurement target included in the acoustic wave image with reference to the acoustic wave image and acoustic wave images captured before and after the acoustic wave image in time series.

4. The acoustic wave diagnostic apparatus according to any of claims 1 to 3,
wherein the measurement execution instruction receiving unit determines freezing the acoustic wave images sequentially displayed on the display unit by the user through the operation unit to be the measurement execution instruction.

5. The acoustic wave diagnostic apparatus according to any of claims 1 to 3,
wherein the measurement execution instruction receiving unit determines displaying the acoustic wave image stored in a memory on the display unit by the user through the operation unit to be the measurement execution instruction.

6. The acoustic wave diagnostic apparatus according to any one of claims 1 to 5,
wherein the measurement target determination unit determines whether or not the measurement target included in the acoustic wave image is singular.

7. The acoustic wave diagnostic apparatus according to claim 6,
wherein the measurement target determination unit determines that the measurement target has not been determined in the acoustic wave image in a case where scores of likelihood assigned to a pattern in the acoustic wave image are equal to or less than a predetermined threshold value.

8. The acoustic wave diagnostic apparatus according to claim 7, further comprising:
a warning unit that issues a warning in a case where it is determined that the measurement target has not been determined by the measurement target determination unit.

9. The acoustic wave diagnostic apparatus according to claim 6,
wherein the measurement target determination unit determines whether the measurement target included in the acoustic wave image is singular or plural.

10. The acoustic wave diagnostic apparatus according to claim 7, further comprising:
a warning unit that issues a warning in a case where it is determined that a plurality of the measurement targets have been determined by the measurement target determination unit,
wherein the warning unit displays the plurality of determined measurement targets in different colors on the display unit.

11. The acoustic wave diagnostic apparatus according to any one of claims 1 to 7, further comprising:
a warning unit that issues a warning in a case where it is determined that the measurement target has not been determined by the measurement target determination unit and a case where it is determined that a plurality of the measurement targets have been determined by the measurement target determination unit; and
a measurement target designation receiving unit that receives designation of a measurement target by the user through the operation unit in a case where a warning is issued by the warning unit,
wherein the measurement unit measures the measurement target received by the measurement target designation receiving unit.

12. The acoustic wave diagnostic apparatus according to claim 11,
wherein the warning unit displays a warning on the display unit and displays a plurality of measurement target candidates on the display unit in a case where it is determined that the measurement target has not been determined by the measurement target determination unit, and
the measurement target designation receiving unit receives designation of a measurement target selected by the user through the operation unit from the plurality of measurement target candidates displayed on the display unit.

13. The acoustic wave diagnostic apparatus according to claim 11,
wherein, in a case where it is determined that a plurality of the measurement targets have been determined by the measurement target determination unit, the warning unit displays a warning on the display unit and displays the plurality of determined measurement targets on the display unit, and
the measurement target designation receiving unit receives designation of a measurement target selected by the user through the operation unit from the plurality of measurement targets displayed on the display unit.

14. The acoustic wave diagnostic apparatus according to any one of the preceding claims,
wherein the measurement unit displays a name of a measurement target that has been measured, a measurement value, and a measurement line used for measurement on the display unit as the measurement result.

15. The acoustic wave diagnostic apparatus according to claim 14, further comprising:
a measurement target correction receiving unit that receives a measurement target correction instruction for the measurement result displayed on the display unit from the user through the operation unit,
wherein the measurement algorithm setting unit sets a measurement algorithm corresponding to a corrected measurement target in a case where the measurement target correction receiving unit receives a measurement target correction instruction, and
the measurement unit measures the corrected measurement target again based on the measurement algorithm set by the measurement algorithm setting unit.

16. The acoustic wave diagnostic apparatus according to claim 14, further comprising:
a measurement line correction receiving unit that receives a measurement line correction instruction for the measurement result displayed on the display unit from the user through the operation unit,
wherein the measurement unit performs measurement again based on the corrected measurement line in a case where the measurement line correction receiving unit receives a measurement line correction instruction.

17. The acoustic wave diagnostic apparatus according to any one of claims 1 to 16,
wherein the acoustic wave image is any one of an ultrasound image, a photoacoustic wave image, or a composite image of an ultrasound image and a photoacoustic wave image.

18. A control method of an acoustic wave diagnostic apparatus, comprising:
displaying an acoustic wave image;
receiving a measurement execution instruction from the user through an operation unit;
determining a measurement target included in the displayed acoustic wave image by using machine learning, the measurement target including an organ or a lesion part;
setting a measurement algorithm based on the determined measurement target;
measuring the measurement target in the acoustic wave image based on the set measurement algorithm; and
displaying a measurement result;
wherein determining a measurement target included in the displayed acoustic wave image by using machine learning comprise assigning a likelihood for learned pattern data to the pattern included in the ultrasound image and performing threshold value determination for the likelihood, the likelihood being a value indicating the likelihood of the pattern included in the ultrasound image for a plurality of pieces of learned pattern data.

## Patentansprüche

1. Akustikwellen-Diagnosevorrichtung, umfassend:
eine Bedienungseinheit (17) für einen Benutzer, um eine Eingabebedienung durchzuführen;
eine Messausführungsanweisungs-Empfangseinheit (15), die eine Messausführungsanweisung von dem Benutzer über die Bedienungseinheit empfängt;
eine Anzeigeeinheit (8), die ein erfasstes Akustikwellenbild anzeigt;
eine Messziel-Bestimmungseinheit (9), die ein in dem auf der Anzeigeeinheit angezeigten Akustikwellenbild enthaltenes Messziel unter Verwendung von maschinellem Lernen bestimmt, wobei das Messziel ein Organ oder einen Läsionsteil enthält;
eine Messalgorithmus-Einstelleinheit (11), die einen Messalgorithmus auf der Grundlage des Messziels einstellt; und
eine Messeinheit (10), die das Messziel in dem Akustikwellenbild auf der Grundlage des von der Messalgorithmus-Einstelleinheit eingestellten Messalgorithmus misst und ein Messergebnis auf der Anzeigeeinheit anzeigt;
wobei die Messziel-Bestimmungseinheit (9) das Messziel unter Verwendung von maschinellem Lernen bestimmt, indem sie eine Wahrscheinlichkeit für erlernte Musterdaten dem in dem Akustikwellenbild enthaltenen Muster zuordnet und Schwellenwertbestimmung für die Wahrscheinlichkeit durchführt, wobei die Wahrscheinlichkeit ein Wert ist, der die Wahrscheinlichkeit des in dem Akustikwellenbild enthaltenen Musters für mehrere erlernte Musterdaten angibt.

2. Akustikwellen-Diagnosevorrichtung nach Anspruch 1,
wobei in einem Fall, in dem bestimmt wird, dass das Muster in dem Akustikwellenbild als mehrere Messziele bestimmt wird, bestimmt wird, dass ein Muster in dem Akustikwellenbild als mehrere Messziele in einem Fall bestimmt wird, in dem die dem Muster für die mehreren Messziele zugeordneten Wahrscheinlichkeiten einen vorbestimmten Schwellenwert überschreiten.

3. Akustikwellen-Diagnosevorrichtung nach einem der Ansprüche 1 bis 2,
wobei die Messziel-Bestimmungseinheit das in dem Akustikwellenbild enthaltene Messziel unter Bezugnahme auf das Akustikwellenbild und Akustikwellenbilder, die vor und nach dem Akustikwellenbild in Zeitreihen aufgenommen worden sind, bestimmt.

4. Akustikwellen-Diagnosevorrichtung nach einem der Ansprüche 1 bis 3,
wobei die Messausführungsanweisungs-Empfangseinheit Einfrieren der auf der Anzeigeeinheit sequentiell angezeigten Akustikwellenbilder durch den Benutzer über die Bedienungseinheit als die Messausführungsanweisung bestimmt.

5. Akustikwellen-Diagnosevorrichtung nach einem der Ansprüche 1 bis 3,
wobei die Messausführungsanweisungs-Empfangseinheit Anzeigen des in einem Speicher gespeicherten Akustikwellenbildes auf der Anzeigeeinheit durch den Benutzer über die Bedienungseinheit als die Messausführungsanweisung bestimmt.

6. Akustikwellen-Diagnosevorrichtung nach einem der Ansprüche 1 bis 5,
wobei die Messziel-Bestimmungseinheit bestimmt, ob das in dem Akustikwellenbild enthaltene Messziel singulär ist oder nicht.

7. Akustikwellen-Diagnosevorrichtung nach Anspruch 6,
wobei die Messziel-Bestimmungseinheit bestimmt, dass das Messziel in dem Akustikwellenbild nicht bestimmt worden ist, in einem Fall, in dem Wahrscheinlichkeitswerte, die einem Muster in dem Akustikwellenbild zugeordnet sind, gleich oder kleiner als ein vorbestimmter Schwellenwert sind.

8. Akustikwellen-Diagnosevorrichtung nach Anspruch 7, ferner umfassend:
eine Warnungseinheit, die eine Warnung in einem Fall ausgibt, in dem bestimmt wird, dass das Messziel von der Messziel-Bestimmungseinheit nicht bestimmt worden ist.

9. Akustikwellen-Diagnosevorrichtung nach Anspruch 6,
wobei die Messziel-Bestimmungseinheit bestimmt, ob das in dem Akustikwellenbild enthaltene Messziel singulär oder plural ist.

10. Akustikwellen-Diagnosevorrichtung nach Anspruch 7, ferner umfassend:
eine Warnungseinheit, die eine Warnung in einem Fall ausgibt, in dem bestimmt wird, dass mehrere der Messziele von der Messziel-Bestimmungseinheit bestimmt worden sind,
wobei die Warnungseinheit die mehreren bestimmten Messziele in unterschiedlichen Farben auf der Anzeigeeinheit anzeigt.

11. Akustikwellen-Diagnosevorrichtung nach einem der Ansprüche 1 bis 7, ferner umfassend:
eine Warnungseinheit, die eine Warnung in einem Fall ausgibt, in dem bestimmt wird, dass das Messziel von der Messziel-Bestimmungseinheit nicht bestimmt worden ist, und einem Fall, in dem bestimmt wird, dass mehrere der Messziele von der Messziel-Bestimmungseinheit bestimmt worden sind; und
eine Messzieldesignations-Empfangseinheit, die Designation eines Messziels von dem Benutzer über die Bedienungseinheit in einem Fall empfängt, in dem eine Warnung von der Warnungseinheit ausgegeben wird,
wobei die Messeinheit das von der Messzieldesignations-Empfangseinheit empfangene Messziel misst.

12. Akustikwellen-Diagnosevorrichtung nach Anspruch 11,
wobei die Warnungseinheit eine Warnung auf der Anzeigeeinheit anzeigt und mehrere Messzielkandidaten auf der Anzeigeeinheit in einem Fall anzeigt, in dem bestimmt wird, dass das Messziel von der Messziel-Bestimmungseinheit nicht bestimmt worden ist, und
die Messzieldesignations-Empfangseinheit Designation eines Messziels, das von dem Benutzer über die Bedienungseinheit ausgewählt worden ist, von den mehreren Messzielkandidaten, die auf der Anzeigeeinheit angezeigt werden, empfängt.

13. Akustikwellen-Diagnosevorrichtung nach Anspruch 11,
wobei in einem Fall, in dem bestimmt wird, dass mehrere der Messziele von der Messziel-Bestimmungseinheit bestimmt worden sind, die Warnungseinheit eine Warnung auf der Anzeigeeinheit anzeigt und die mehreren bestimmten Messziele auf der Anzeigeeinheit anzeigt, und
die Messzieldesignations-Empfangseinheit Designation eines Messziels, das von dem Benutzer über die Bedienungseinheit ausgewählt worden ist, von den mehreren Messzielen, die auf der Anzeigeeinheit angezeigt werden, empfängt.

14. Akustikwellen-Diagnosevorrichtung nach einem der vorhergehenden Ansprüche,
wobei die Messeinheit einen Namen eines Messziels, das gemessen worden ist, einen Messwert und eine Messlinie, die zur Messung verwendet wird, auf der Anzeigeeinheit als das Messergebnis anzeigt.

15. Akustikwellen-Diagnosevorrichtung nach Anspruch 14, ferner umfassend:
eine Messzielkorrektur-Empfangseinheit, die eine Messzielkorrekturanweisung für das auf der Anzeigeeinheit angezeigte Messergebnis von dem Benutzer über die Bedienungseinheit empfängt,
wobei die Messalgorithmus-Einstelleinheit einen Messalgorithmus, der einem korrigierten Messziel entspricht, in einem Fall einstellt, in dem die Messzielkorrektur-Empfangseinheit eine Messzielkorrekturanweisung empfängt, und
die Messeinheit das korrigierte Messziel erneut auf der Grundlage des von der Messalgorithmus-Einstelleinheit eingestellten Messalgorithmus misst.

16. Akustikwellen-Diagnosevorrichtung nach Anspruch 14, ferner umfassend:
eine Messlinienkorrektur-Empfangseinheit, die eine Messlinienkorrekturanweisung für das auf der Anzeigeeinheit angezeigte Messergebnis von dem Benutzer über die Bedienungseinheit empfängt,
wobei die Messeinheit in einem Fall, in dem die Messlinienkorrektur-Empfangseinheit eine Messlinienkorrekturanweisung empfängt, Messung erneut auf der Grundlage der korrigierten Messlinie durchführt.

17. Akustikwellen-Diagnosevorrichtung nach einem der Ansprüche 1 bis 16,
wobei das Akustikwellenbild ein Ultraschallbild, ein photoakustisches Wellenbild oder ein zusammengesetztes Bild eines Ultraschallbildes und eines photoakustischen Wellenbildes ist.

18. Steuerverfahren einer Akustikwellen-Diagnosevorrichtung, umfassend:
Anzeigen eines Akustikwellenbildes;
Empfangen einer Messausführungsanweisung von dem Benutzer über eine Bedienungseinheit;
Bestimmen eines in dem angezeigten Akustikwellenbild enthaltenen Messziels unter Verwendung von maschinellem Lernen, wobei das Messziel ein Organ oder einen Läsionsteil enthält;
Einstellen eines Messalgorithmus auf der Grundlage des bestimmten Messziels;
Messen des Messziels in dem Akustikwellenbild auf der Grundlage des eingestellten Messalgorithmus; und
Anzeigen eines Messergebnisses;
wobei Bestimmen eines in dem angezeigten Akustikwellenbild enthaltenen Messziels unter Verwendung von maschinellem Lernen Zuordnen einer Wahrscheinlichkeit für erlernte Musterdaten zu dem in dem Ultraschallbild enthaltenen Muster und Durchführen von Schwellenwertbestimmung für die Wahrscheinlichkeit umfasst, wobei die Wahrscheinlichkeit ein Wert ist, der die Wahrscheinlichkeit des in dem Ultraschallbild enthaltenen Musters für mehrere erlernte Musterdaten angibt.

## Revendications

1. Appareil de diagnostic par ondes acoustiques comprenant :
une unité d'opération (17) permettant à un utilisateur d'effectuer une opération d'entrée ;
une unité de réception d'instruction d'exécution de mesure (15) qui reçoit une instruction d'exécution de mesure de l'utilisateur par le biais de l'unité d'opération ;
une unité d'affichage (8) qui affiche une image d'onde acoustique acquise ;
une unité de détermination de cible de mesure (9) qui détermine une cible de mesure incluse dans l'image d'onde acoustique affichée sur l'unité d'affichage en utilisant un apprentissage automatique, la cible de mesure incluant un organe ou une partie lésée ;
une unité de réglage d'algorithme de mesure (11) qui règle un algorithme de mesure sur la base de la cible de mesure ; et
une unité de mesure (10) qui mesure la cible de mesure dans l'image d'onde acoustique sur la base de l'algorithme de mesure réglé par l'unité de réglage d'algorithme de mesure et affiche un résultat de mesure sur l'unité d'affichage ;
dans lequel l'unité de détermination de cible de mesure (9) détermine la cible de mesure en utilisant un apprentissage automatique en attribuant une vraisemblance pour des données de motif apprises au motif inclus dans l'image d'onde acoustique et en effectuant une détermination de valeur seuil pour la vraisemblance, la vraisemblance étant une valeur indiquant la vraisemblance du motif inclus dans l'image d'onde acoustique pour une pluralité d'éléments de données de motif apprises.

2. Appareil de diagnostic par ondes acoustiques selon la revendication 1,
dans lequel, dans un cas où il est déterminé que le motif dans l'image d'onde acoustique est déterminé comme une pluralité de cibles de mesure, il est déterminé qu'un motif dans l'image d'onde acoustique est déterminé comme une pluralité de cibles de mesure dans un cas où les vraisemblances attribuées au motif pour la pluralité de cibles de mesure dépassent une valeur seuil prédéterminée.

3. Appareil de diagnostic par ondes acoustiques selon l'une quelconque des revendications 1 à 2,
dans lequel l'unité de détermination de cible de mesure détermine la cible de mesure incluse dans l'image d'onde acoustique en se référant à l'image d'onde acoustique et à des images d'ondes acoustiques capturées avant et après l'image d'onde acoustique dans une série temporelle.

4. Appareil de diagnostic par ondes acoustiques selon l'une quelconque des revendications 1 à 3,
dans lequel l'unité de réception d'instruction d'exécution de mesure détermine que le gel des images d'ondes acoustiques affichées séquentiellement sur l'unité d'affichage par l'utilisateur par le biais de l'unité d'opération est l'instruction d'exécution de mesure.

5. Appareil de diagnostic par ondes acoustiques selon l'une quelconque des revendications 1 à 3,
dans lequel l'unité de réception d'instruction d'exécution de mesure détermine que l'affichage de l'image d'onde acoustique stockée dans une mémoire sur l'unité d'affichage par l'utilisateur par le biais de l'unité d'opération est l'instruction d'exécution de mesure.

6. Appareil de diagnostic par ondes acoustiques selon l'une quelconque des revendications 1 à 5,
dans lequel l'unité de détermination de cible de mesure détermine si oui ou non la cible de mesure incluse dans l'image d'onde acoustique est unique.

7. Appareil de diagnostic par ondes acoustiques selon la revendication 6,
dans lequel l'unité de détermination de cible de mesure détermine que la cible de mesure n'a pas été déterminée dans l'image d'onde acoustique dans un cas où des scores de vraisemblance attribués à un motif dans l'image d'onde acoustique sont égaux ou inférieurs à une valeur seuil prédéterminée.

8. Appareil de diagnostic par ondes acoustiques selon la revendication 7, comprenant en outre :
une unité d'avertissement qui émet un avertissement dans un cas où il est déterminé que la cible de mesure n'a pas été déterminée par l'unité de détermination de cible de mesure.

9. Appareil de diagnostic par ondes acoustiques selon la revendication 6,
dans lequel l'unité de détermination de cible de mesure détermine si la cible de mesure incluse dans l'image d'onde acoustique est unique ou multiple.

10. Appareil de diagnostic par ondes acoustiques selon la revendication 7, comprenant en outre :
une unité d'avertissement qui émet un avertissement dans un cas où il est déterminé qu'une pluralité des cibles de mesure ont été déterminées par l'unité de détermination de cible de mesure,
dans lequel l'unité d'avertissement affiche la pluralité de cibles de mesure déterminées en couleurs différentes sur l'unité d'affichage.

11. Appareil de diagnostic par ondes acoustiques selon l'une quelconque des revendications 1 à 7, comprenant en outre :
une unité d'avertissement qui émet un avertissement dans un cas où il est déterminé que la cible de mesure n'a pas été déterminée par l'unité de détermination de cible de mesure et un cas où il est déterminé qu'une pluralité des cibles de mesure ont été déterminées par l'unité de détermination de cible de mesure ; et
une unité de réception de désignation de cible de mesure qui reçoit la désignation d'une cible de mesure par l'utilisateur par le biais de l'unité d'opération dans un cas où un avertissement est émis par l'unité d'avertissement,
dans lequel l'unité de mesure mesure la cible de mesure reçue par l'unité de réception de désignation de cible de mesure.

12. Appareil de diagnostic par ondes acoustiques selon la revendication 11,
dans lequel l'unité d'avertissement affiche un avertissement sur l'unité d'affichage et affiche une pluralité de candidats de cible de mesure sur l'unité d'affichage dans un cas où il est déterminé que la cible de mesure n'a pas été déterminée par l'unité de détermination de cible de mesure, et
l'unité de réception de désignation de cible de mesure reçoit la désignation d'une cible de mesure sélectionnée par l'utilisateur par le biais de l'unité d'opération parmi la pluralité de candidats de cible de mesure affichés sur l'unité d'affichage.

13. Appareil de diagnostic par ondes acoustiques selon la revendication 11,
dans lequel, dans un cas où il est déterminé qu'une pluralité des cibles de mesure ont été déterminées par l'unité de détermination de cible de mesure, l'unité d'avertissement affiche un avertissement sur l'unité d'affichage et affiche la pluralité de cibles de mesure déterminées sur l'unité d'affichage, et
l'unité de réception de désignation de cible de mesure reçoit la désignation d'une cible de mesure sélectionnée par l'utilisateur par le biais de l'unité d'opération parmi la pluralité de cibles de mesure affichées sur l'unité d'affichage.

14. Appareil de diagnostic par ondes acoustiques selon l'une quelconque des revendications précédentes,
dans lequel l'unité de mesure affiche un nom d'une cible de mesure qui a été mesurée, une valeur de mesure et une ligne de mesure utilisée pour la mesure sur l'unité d'affichage en tant que le résultat de mesure.

15. Appareil de diagnostic par ondes acoustiques selon la revendication 14, comprenant en outre :
une unité de réception de correction de cible de mesure qui reçoit de l'utilisateur, par le biais de l'unité d'opération, une instruction de correction de cible de mesure pour le résultat de mesure affiché sur l'unité d'affichage,
dans lequel l'unité de réglage d'algorithme de mesure règle un algorithme de mesure correspondant à une cible de mesure corrigée dans un cas où l'unité de réception de correction de cible de mesure reçoit une instruction de correction de cible de mesure, et
l'unité de mesure mesure à nouveau la cible de mesure corrigée sur la base de l'algorithme de mesure réglé par l'unité de réglage d'algorithme de mesure.

16. Appareil de diagnostic par ondes acoustiques selon la revendication 14, comprenant en outre :
une unité de réception de correction de ligne de mesure qui reçoit de l'utilisateur par le biais de l'unité d'opération, une instruction de correction de ligne de mesure pour le résultat de mesure affiché sur l'unité d'affichage,
dans lequel l'unité de mesure effectue à nouveau une mesure sur la base de la ligne de mesure corrigée dans un cas où l'unité de réception de correction de ligne de mesure reçoit une instruction de correction de ligne de mesure.

17. Appareil de diagnostic par ondes acoustiques selon l'une quelconque des revendications 1 à 16,
dans lequel l'image d'onde acoustique est l'une quelconque d'une image ultrasonore, d'une image d'onde photoacoustique ou d'une image composite d'une image ultrasonore et d'une image d'onde photoacoustique.

18. Procédé de contrôle d'un appareil de diagnostic par ondes acoustiques, comprenant :
afficher une image d'onde acoustique ;
recevoir une instruction d'exécution de mesure de l'utilisateur par le biais d'une unité d'opération ;
déterminer une cible de mesure incluse dans l'image d'onde acoustique affichée en utilisant un apprentissage automatique, la cible de mesure incluant un organe ou une partie lésée ;
régler un algorithme de mesure sur la base de la cible de mesure déterminée ;
mesurer la cible de mesure dans l'image d'onde acoustique sur la base de l'algorithme de mesure réglé ; et
afficher un résultat de mesure ;
dans lequel la détermination d'une cible de mesure incluse dans l'image d'onde acoustique affichée en utilisant un apprentissage automatique comprend l'attribution d'une vraisemblance pour des données de motif apprises au motif inclus dans l'image ultrasonore et la réalisation d'une détermination de valeur seuil pour la vraisemblance, la vraisemblance étant une valeur indiquant la vraisemblance du motif inclus dans l'image ultrasonore pour une pluralité d'éléments de données de motif apprises.
